# DEMANDE DE BREVET EUROPEEN

(11) **EP 0 852 222 A1**
(43) Date de publication de la demande: **08.07.1998**
(21) Numéro de dépôt: 98400011.7
(22) Date de dépôt: 06.01.1998
(51) Int. Cl.: C02F 1/28, A61L 2/02, B01D 39/06, B01D 39/20, B01D 15/00

(54) **Procédés et dispositifs pour la désinfection et le recyclage d'un effluent aqueux**

(30) Priorité: 06.01.1997 FR 9700064
(71) Demandeur: Cultilene BV, 2713 HG Zoetermeer (NL)
(72) Inventeur: De Graaf, Martien, 2141 CN Vijfhuizen (NL); Kafka, Bernard, Auvillers, 60290 Rantigny (FR); Kashiwakura, Maki, 94300 Vincennes (FR)
(74) Mandataire: Muller, René

(57) **Abrégé**

Selon l'invention, on désinfecte un effluent aqueux acide (5), tel que de l'eau de drainage d'une culture hors-sol (1) ou en pot, qui renferme des micro-organismes pathogènes de ladite culture, en vue de son recyclage en tant que liquide d'arrosage et/ou nutritif pour ladite culture, en faisant circuler l'effluent (5) à travers une masse de matériau filtrant (18) à base de laine de verre.

## Description

La présente invention se rapporte au traitement d'effluents liquides aqueux, tels que des eaux de drainage de culture hors-sol ou de culture de plantes en pot.

Dans ce type de cultures, où les plants végétaux sont cultivés sur des substrats minéraux ou organiques de dimensions limitées, l'écoulement par gravité de l'eau d'arrosage non absorbée par la plante est source de volumes importants d'effluents liquides qui renferment quantité d'éléments entraînés par l'eau écoulée. S'agissant de cultures de type hors-sol, ces effluents aqueux correspondent à l'excès de solution nutritive non retenu par le substrat. En raison de leur teneur en éléments nutritifs, principalement constitués de sels minéraux notamment de nitrates, leur rejet dans la nature peut être nuisible à l'environnement, et représente en outre une perte notable de matière première. Il serait donc intéressant à la fois d'un point de vue écologique et économique, de pouvoir recycler ces effluents dans le circuit d'arrosage et/ou d'alimentation en solution nutritive des cultures.

Malheureusement, même dans le cas de culture hors-sol sur substrat minéral, le milieu de culture est le siège du développement de pathogènes affectant principalement les racines des plantes, tels que des champignons, des bactéries, des virus ou des nématodes. La recirculation des eaux de drainage des cultures entraînerait donc une concentration de pathogènes sur le site de culture, et en conséquence un risque élevé de voir les maladies contaminer toute la culture. Pour éviter cela, une désinfection préalable des eaux de drainage s'impose.

Les méthodes traditionnelles mettant en oeuvre des agents désinfectants chimiques ne peuvent être utilisées et il faut recourir à des techniques plus douces telles qu'un traitement thermique, un traitement par l'ozone ou traitement sous rayonnement ultra-violet. Ces techniques ont l'inconvénient d'être très coûteuses et ne sont pas rentables pour de petites exploitations, notamment de moins de 1 hectare.

En outre, elles ne sont pas toujours adaptées au traitement de solutions contenant des sels minéraux. Ainsi, par exemple, un traitement thermique à 90-100°C, même pendant une courte durée de l'ordre de 30 secondes, fait précipiter certains sels minéraux, notamment les sels de calcium, et il faut donc prévoir des opérations supplémentaires de traitement pour séparer les précipités et rééquilibrer la composition de l'effluent avant de recycler ce dernier.

Une autre méthode de purification connue est la filtration lente sur sable. Elle consiste à faire circuler l'effluent aqueux de haut en bas à faible débit, à travers une colonne de sable dont la granulométrie peut varier entre 0,15 et 1,6 mm, de préférence de sable fin de granulométrie comprise entre 0,15 et 0,35 mm. Appliquée à la désinfection d'eaux de drainage de cultures hors-sol, cette méthode ne s'est révélée efficace que pour arrêter les pathogènes de grande taille. Elle requiert en outre un volume de sable très élevé ce qui rend les installations de filtration très encombrantes. Par ailleurs, l'entretien des colonnes de sable est assez contraignant : après quelques semaines d'utilisation, on observe dans la partie supérieure de la colonne le développement d'algues qui obturent progressivement l'espace intergranulaire et perturbent le passage de l'eau dans la colonne. La couche supérieure de sable doit donc être périodiquement renouvelée, ce qui n'est pas sans poser quelques difficultés techniques car le sable est délicat à manipuler.

Le document FR-A-2 362 791 décrit un procédé pour purifier de l'eau telle que l'eau des aquariums et similaires, qui contient des microorganismes, consistant à filtrer l'eau sur une substance de type inerte, fibreuse, de préférence de la laine roche, avec un débit élevé supérieur à environ 1-1,5 mètre par heure (m³/m².h).

Pour l'élimination de microorganismes de petite taille tels que bactéries pathogènes et microbes, le procédé nécessite cependant l'oxygénation de l'eau à saturation, préalablement à ou pendant la filtration.

L'utilisation de laine de roche notamment sous forme granulaire pose en outre des problèmes d'homogénéité de la filtration liés à la présence de courts-circuits de filtration en périphérie du matériau filtrant. Pour résoudre ce problème WO-A-96 38387 décrit un filtre sous forme de rouleau de laine minérale disposé verticalement dans une cuve, l'espace entre le rouleau et la paroi de la cuve étant comblé par un matériau capable de gonfler dans l'eau tel qu'un polymère polyacrylamide.

Cette solution souffre d'une certaine complexité de réalisation, en particulier lorsque plusieurs rouleaux doivent être installés dans une cuve de grande contenance. En outre, il se présente des cas où cette disposition ne suffit pas pour obtenir avec de la laine de roche une filtration assez homogène pour le degré d'épuration souhaité. C'est le cas notamment lorsque l'eau traitée présente un pH très faible.

La présente invention a pour but d'obvier à ces divers inconvénients. Plus particulièrement, la présente invention a pour but de fournir une méthode de désinfection d'effluents aqueux, tels que des eaux de drainage de culture, qui soit peu coûteuse, efficace sur un large spectre de pathogènes y compris pour des effluents très acides, simple à mettre en oeuvre et qui soit compatible avec des exploitations de petite comme de grande taille.

De façon inattendue, les présents inventeurs ont constaté que les objectifs ci-dessus peuvent être atteints, et notamment que la technique de la filtration était efficace vis-à-vis de nombreux pathogènes en milieu acide, en choisissant, en tant que matériau filtrant un matériau fibreux à base de laine de verre.

A cet égard, l'invention a pour premier objet un procédé de désinfection d'un effluent aqueux acide, notamment de l'eau de drainage d'une culture hors-sol ou d'une culture de plante en pot qui renferme des micro-organismes pathogènes de ladite culture, en vue de son recyclage dans le circuit d'arrosage et/ou d'alimentation en liquide nutritif de ladite culture, caractérisé en ce que l'étape de désinfection consiste à faire circuler l'effluent à travers une masse de matériau filtrant à base de laine de verre.

S'agissant du matériau filtrant dans la présente demande, on entend par « à base de laine de verre», un matériau constitué essentiellement de laine de verre, renfermant éventuellement les additifs usuels de fabrication et mise en forme des laines minérales tels que ensimage ou liant, ou des additifs destinés à ajuster le degré d'hydrophilie du matériau, tels que des agents mouillants ou tensio-actifs. Ne sont pas compris dans ces additifs les agents actifs désinfectants, notamment bactéricides, virucides ou fongicides, destinés à éliminer les microorganismes par action chimique.

Selon l'invention, l'effluent est filtré à travers une masse de matériau filtrant. Par « masse » on entend dans la présente description que le matériau filtrant est tel que l'effluent traverse une épaisseur non négligeable de laine minérale.

La nature fibreuse du matériau filtrant selon l'invention s'est révélée déterminante pour la qualité de la filtration. L'enchevêtrement de la laine dans le matériau impose à l'effluent un parcours long et sinueux pour traverser le matériau suivant de nombreuses lignes de courant. De cette façon, l'effluent traité est exposé à une plus grande surface filtrante que dans le cas de matériaux granulaires comme le sable, et les microorganismes sont retenus avec une plus grande efficacité dans les pores du matériau.

Les laines de verre utilisables selon l'invention peuvent être choisies parmi tous les matériaux habituellement désignés par ce terme, qui regroupe de façon générale tous les matériaux susceptibles d'être obtenus par fibrage d'une composition minérale fortement silicatée à l'état fondu. Il a été observé, en utilisant selon l'invention de la laine de verre comme média filtrant pour effluents aqueux, que ce matériau a une remarquable aptitude à retenir les microorganismes, semble-t-il grâce à une affinité élevée de ces derniers pour la laine de verre en milieu acide, et est capable de conserver ces propriétés à long terme en milieu acide, grâce à des caractéristiques mécaniques et physico-chimiques très avantageuses.

Sa haute résistance en milieu acide rend la laine de verre tout particulièrement adaptée au traitement d'effluents aqueux acides, notamment de pH compris entre 4 et 7, plus particulièrement inférieur ou égal à 6,5, tels que des eaux de drainage de culture hors-sols dont le pH peut atteindre des valeurs très faibles de l'ordre de 3 à 5, de façon permanente ou bien seulement de façon momentanée (ponctuelle) au cours de la culture, selon les paramètres de cette dernière.

De préférence, le procédé de désinfection opère par filtration lente sur le matériau à base de laine minérale, en faisant circuler l'effluent à travers le matériau filtrant à une vitesse linéique avantageusement inférieure à 0,3 m/h, de préférence à une vitesse linéique d'environ 0,1 m/h. L'efficacité de la désinfection est d'autant plus grande que la vitesse de l'effluent est faible.

De façon très avantageuse, le matériau filtrant a une surface spécifique élevée, notamment d'au moins 0,01 m²/cm³, de préférence au moins 0,012 m²/cm³

Des surfaces spécifiques satisfaisantes sont avantageusement obtenues avec un matériau filtrant renfermant une laine de verre caractérisée par un micronaire inférieur à 7 pour 5 grammes, notamment de 1 à 5.

La mesure du micronaire appelée aussi « indice de finesse » rend compte de la surface spécifique grâce à la mesure de la perte de charge aérodynamique lorsqu'une quantité donnée de laine extraite d'un matelas non ensimé est soumise à une pression donnée d'un gaz - en général de l'air ou de l'azote. Cette mesure est usuelle dans les unités de production de laines minérales, elle est normalisée (DIN 53941 ou ASTM D 1448) et elle utilise un appareil dit « appareil micronaire », tel qu'une machine SHEFFIELD, type FAM 60 P. Cette machine comporte une arrivée d'air (ou d'azote) sous pression, une vanne de réglage de cette pression, un débitmètre, une chambre cylindrique à axe vertical avec arrivée des gaz en partie inférieure. La laine pesée, (le plus souvent, 5 grammes ± 0,01 g) est pressée au fond de la chambre par un bouchon calibré qui laisse échapper les gaz. Un essai préliminaire permet d'ajuster le débit d'air à une valeur donnée, toujours la même avant de commencer l'essai du tampon de laine. La mesure du micronaire ou de l'indice de finesse, consiste à relever l'indication du débitmètre normalisé, lorsque la laine est en place. Pour travailler dans la même gamme de pertes de charge, il est nécessaire d'adapter la quantité de laine testée en diminuant la masse lorsque le diamètre diminue. C'est pourquoi l'on mentionne celle-ci en même temps que le résultat du débit.

Le matériau filtrant peut se présenter sous des formes diverses, sous réserve que la laine de verre soit arrangée de façon à ménager des pores dans la masse du matériau. Ainsi, on peut utiliser notamment de la laine minérale en vrac ou bien sous forme de feutre, et ce de manière plus ou moins dense. Sous une forme ou sous une autre, la laine minérale utilisée a de préférence une masse volumique de 30 à 250 kg/m³, notamment d'environ 50 à 200 kg/m³, de préférence de l'ordre de 50 à 100 kg/m³. Il semble néanmoins que l'élimination des microorganismes soit d'autant plus efficace que la densité est élevée. Ainsi, pour un effluent donné, un filtre de 50 kg/m³ de densité est légèrement moins efficace qu'un filtre de 100 kg/m³.

Dans un mode de réalisation préféré, au moins une partie du matériau filtrant est constituée de laine de verre sous forme de feutre (y compris les produits dits mats ou matelas) dont la cohésion est assurée par un liant approprié. Un feutre disposé à l'extrémité de sortie du filtre permet avantageusement d'arrêter les fibres libres éventuellement entraînées par l'effluent filtré.

Un premier avantage de ces feutres réside dans leur stabilité mécanique : bien que ponctuelle, la liaison entre les fibres est suffisamment résistante pour que la structure d'un tel feutre ne soit pas modifiée par le passage du liquide à filtrer. Contrairement à ce que l'on observe avec le sable, il ne se forme pas dans ces feutres de canaux susceptibles de court-circuiter la filtration. En complément de cette stabilité mécanique, ces feutres de laine de verre sont dotés d'une certaine élasticité qui les rend avantageux en particulier par rapport aux produits plus rigides à base de laine de roche, notamment lors de la mise en place dans un contenant tel qu'une colonne de filtration. On utilise d'ailleurs avantageusement pour garnir des colonnes de filtration mettant en oeuvre le procédé selon l'invention des blocs de feutre ou de matelas découpés à des dimensions supérieures aux dimensions intérieures de la colonne (la différence pouvant représenter par exemple de 1 à 5 %, notamment de l'ordre de 3 % desdites dimensions intérieures).

Dans une variante avantageuse, au moins une partie du matériau filtrant renferme un agent mouillant destiné à augmenter le caractère hydrophile de la laine. L'amélioration de la mouillabilité par l'eau du matériau filtrant favorise la diffusion de l'effluent dans tout le volume du filtre et contribue à augmenter l'efficacité de la filtration. De préférence, la partie renfermant l'agent mouillant constitue l'extrémité d'entrée du filtre : le mouillage initial de la laine favorise la diffusion uniforme du liquide dans le reste du matériau. L'agent mouillant peut être choisi parmi les tensioactifs connus en soi, notamment les tensio-actifs non ioniques, de préférence peu moussants.

La quantité de matériau filtrant à utiliser peut être choisie de façon connue en soi par le spécialiste, en fonction notamment du type d'effluent et/ou du débit à traiter, pour atteindre l'épuration désirée. Typiquement, on parvient à éliminer de l'ordre d'au moins 99 % des pathogènes initialement présents dans l'effluent.

Pour la mise en oeuvre d'un procédé de désinfection tel qu'il vient d'être décrit, l'invention a encore pour objet un dispositif pour la désinfection d'un effluent aqueux acide, notamment de l'eau de drainage d'une culture hors-sol ou d'une culture de plante en pot qui renferme des micro-organismes pathogènes de ladite culture caractérisé en ce qu'il comprend :
- une capacité de stockage de l'effluent aqueux,
- une masse de matériau filtrant à base de laine de verre avec une extrémité d'entrée alimentée en effluent aqueux par des moyens d'amenée de liquide reliés à la capacité de stockage et une extrémité de sortie délivrant un effluent désinfecté, et
- des moyens pour faire circuler l'effluent entre lesdites extrémités d'entrée et de sortie.

De façon avantageuse, ladite extrémité d'entrée est située dans la partie supérieure du matériau filtrant et ladite extrémité de sortie est située dans la partie inférieure du matériau filtrant, l'effluent s'écoulant par gravité entre ces deux extrémités, éventuellement sous l'action complémentaire d'une surpression, d'une pompe ou de tout autre moyen pour mettre un liquide en circulation. Cette direction verticale de filtration permet un passage régulier de l'effluent sur toute la section du matériau filtrant et est un facteur qui contribue à l'efficacité de l'épuration.

Ainsi, le matériau filtrant est de préférence disposé verticalement, avantageusement, sur une épaisseur d'au moins 80 cm.

Dans un mode de réalisation avantageux, le matériau filtrant est disposé verticalement, avec l'extrémité d'entrée à un niveau supérieur par rapport à l'extrémité de sortie, et lesdits moyens pour faire circuler l'effluent entre ces deux extrémités du matériau filtrant comprennent une capacité remplie dudit effluent et qui surmonte le matériau filtrant. Avantageusement, cette capacité renferme une colonne de liquide d'une hauteur de 20 à 150 cm.

Comme indiqué précédemment, on peut utiliser la laine de verre sous diverses formes dans le dispositif selon l'invention. Une structure préférée de matériau filtrant, qui est aussi un objet de l'invention, est un filtre à base de laine de verre qui comprend une partie supérieure où la laine de verre est sous forme de feutre, une partie intermédiaire à base de laine de verre, et une partie inférieure éventuellement constituée d'un autre matériau poreux.

La partie intermédiaire peut être constituée de laine de verre en vrac ou bien, elle aussi, d'un feutre de laine de verre, qui peut former une couche distincte de ladite partie supérieure ou bien simplement prolonger cette partie supérieure.

Le matériau poreux formant la partie inférieure peut être quelconque, et sert en particulier à arrêter d'éventuelles fibres de verre entraînées par le filtrat, notamment lorsque la couche intermédiaire est constituée de laine de verre en vrac. Cette fonction peut être remplie par du feutre de laine de verre mais d'autres exemples de matériaux utilisables sont notamment le gravier et le sable.

De préférence, notamment pour la commodité d'emploi, la totalité du filtre est constituée d'un feutre de laine de verre, avantageusement disposé de façon à orienter les fibres transversalement par rapport à la direction de filtration.

A l'issue de l'étape de filtration, dans un dispositif du type qui vient d'être décrit, l'effluent aqueux a une teneur en pathogènes minime et peut être réutilisé pour alimenter une culture en eau, et le cas échéant en éléments nutritifs, sans augmenter le risque que la culture développe la ou les maladies correspondantes.

Ainsi, l'invention a également pour objet un procédé de recyclage d'une eau de drainage acide d'une culture hors-sol ou d'une culture de plante en pot, caractérisé en ce qu'il comprend les étapes consistant à :
- recueillir l'eau de drainage à partir du milieu de culture,
- soumettre l'effluent liquide constitué par cette eau de drainage à un procédé de désinfection sur un matériau filtrant tel que décrit ci-dessus, et
- introduire l'effluent désinfecté récupéré en sortie du matériau filtrant, dans le milieu de culture en tant que liquide d'arrosage et/ou liquide nutritif.

Le cas échéant, lorsque l'eau de drainage a une composition trop pauvre en un élément donné, ou bien un pH trop éloigné du pH du milieu de culture, le procédé de recyclage peut comprendre en outre une étape intermédiaire consistant à réajuster la composition (teneur en sels minéraux, pH...) de l'effluent issu du procédé de désinfection, avant l'étape finale où l'on réintroduit l'effluent désinfecté dans le milieu de culture.

L'invention a également pour objet un dispositif de recyclage d'une eau de drainage acide d'une culture hors-sol ou d'une culture de plante en pot équipée d'un circuit d'arrosage et/ou d'alimentation en solution nutritif, caractérisé en ce qu'il comprend :
- des moyens pour recueillir ladite eau de drainage, reliés à des premiers moyens d'amenée de liquide,
- un dispositif de désinfection tel que décrit ci-dessus, dont la capacité de stockage est alimentée en effluent liquide par lesdits premiers moyens d'amenée de liquide, et
- des seconds moyens d'amenée de liquide reliant l'extrémité de sortie du matériau filtrant audit circuit d'arrosage et/ou d'alimentation en solution nutritive.

D'autres particularités et avantages de l'invention apparaîtront dans la description détaillée qui va suivre faite en regard des dessins annexés sur lesquels :
- la **figure 1** représente une installation de culture hors-sol équipée d'un dispositif de recyclage de l'eau de drainage conforme à l'invention,
- la **figure 2** représente l'évolution du pH dans un matériau filtrant à base de laine de verre selon l'invention en présence d'un effluent acide et, à titre de comparaison dans un matériau à base de laine de roche dans les mêmes conditions.

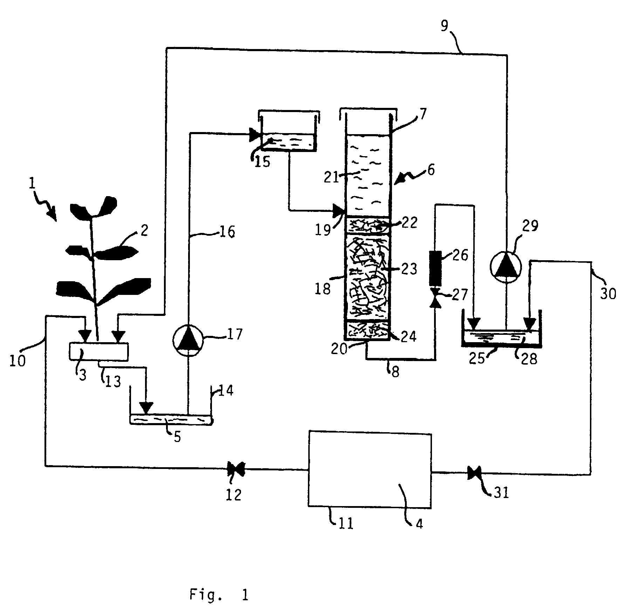

L'installation représentée sur la figure 1 comprend essentiellement une unité 1 de culture hors sol, dans laquelle des plantes 2 sont cultivées sur un substrat 3 minéral ou organique, avec un apport de solution nutritive 4, dont une partie est absorbée par la plante et l'excédent s'écoule hors du substrat 3 sous forme d'eau de drainage 5, et un dispositif 6 de désinfection de l'eau de drainage 5 comprenant principalement une colonne de filtration 7 et des moyens d'amenée de liquide 8, 9 recyclant le filtrat récupéré en pied de la colonne 7 vers l'unité de culture 1. Cette installation fonctionne de la façon suivante.

Dans un exemple particulier d'unité 1 de culture hors-sol, les plantes 2 sont portées par un substrat 3 à base de laine minérale, typiquement de laine de verre ou de roche sous forme de matelas ou de feutre, tel que décrit notamment dans FR-A-2 581 503 et WO-A-89 01 736. L'apport en éléments nutritifs se fait en continu ou en discontinu, par une canalisation 10 reliée à une réserve 11 contenant une solution aqueuse 4 renfermant principalement des sels minéraux tels que nitrates, phosphates... Une vanne 12 est disposée sur la canalisation 10 pour réguler l'apport en solution nutritive 4. Un système de collecte des eaux de drainage est prévu, qui comprend au moins une canalisation 13 débouchant dans un bac de stockage 14.

Que le substrat de culture 3 soit organique ou minéral, le développement des plantes 2 s'accompagne du développement de micro-organismes pathogènes. La plupart de ces pathogènes affectent les racines des plantes, tels que des champignons, virus ou nématodes. Ces pathogènes se retrouvent en quantité non négligeable dans l'eau de drainage 5. C'est pourquoi, il n'est pas envisageable de renvoyer cette eau de drainage 5 telle quelle dans le circuit d'alimentation en solution nutritive, en dépit de sa teneur résiduelle importante en éléments nutritifs, car cela reviendrait à inoculer les pathogènes en continu dans la plante 2.

Pour permettre le recyclage de l'eau de drainage en tant que liquide d'arrosage (apport en eau) ou en tant que liquide nutritif (apport en sels minéraux), l'installation est munie du dispositif de désinfection 6. Ce dispositif comprend un premier réservoir 15 de liquide de grande capacité, dans lequel l'eau de drainage 5 est transférée depuis le bac 14 par l'intermédiaire d'une conduite 16 sur laquelle est installée une pompe 17. Ce réservoir alimente la colonne de filtration 7 verticale qui renferme un matériau filtrant 18 à base de laine de verre et qui comporte une alimentation 19 en liquide à traiter située en amont (au-dessus) du filtre 18 et une évacuation 20 du filtrat située en aval (en-dessous) du filtre 18.

Le matériau filtrant 18 est disposé dans la partie inférieure de la colonne 7, la partie supérieure étant destinée, en fonctionnement, à être remplie de liquide à traiter pour réguler la force motrice qui régit le passage de l'effluent à travers le matériau filtrant. Les dimensions de la colonne 7 et les hauteurs respectives occupées par le matériau filtrant 18 et la colonne d'eau 21 sont variables en fonction principalement de l'importance de l'unité de culture hors-sol 1, par conséquent du débit d'eau de drainage produit.

Pour obtenir une désinfection satisfaisante, il est préférable que l'effluent traverse une certaine épaisseur de matériau filtrant, avantageusement au moins 80 cm. La hauteur de la colonne de liquide 21 surmontant le matériau filtrant dépend essentiellement du débit d'alimentation en eau de drainage. Elle peut varier notamment de 20 à 150 cm. De manière générale, pour des raisons de commodité d'installation, la hauteur de la colonne 7 peut être comprise dans la gamme de 1 à 4 m.

Dans le mode de réalisation représenté sur la figure unique, qui correspond à un mode préféré, le matériau filtrant 18 est constitué de trois couches superposées : une couche supérieure 22 de feutre de laine de verre d'une densité de 50 kg/m³, une couche intermédiaire 23 de feutre de laine de verre d'une densité de 100 kg/m³ et une couche inférieure 24 de feutre de laine de verre d'une densité de 50 kg/m³. De préférence, la couche supérieure 22, et éventuellement la couche inférieure 24, renferme un agent mouillant, par exemple de type non ionique pour faciliter la circulation de l'effluent au sein du matériau filtrant.

A l'extrémité de sortie 20 de la colonne 7, l'eau de drainage désinfectée est recyclée dans le circuit d'alimentation en eau de la culture via les conduites 8 et 9. La conduite 8 achemine le filtrat qui s'écoule à l'extrémité 20 de la colonne vers un bac de rétention 25. Un débitmètre 26 et une vanne 27, installés sur la conduite 8, permettent de contrôler et de réguler le débit de filtrat recyclé. Le bac 25 peut jouer un simple rôle de réserve à partir de laquelle le liquide 28 contenu est acheminé vers l'unité de culture 1 par l'intermédiaire de la pompe 29 et de la conduite 9.

Dans la variante représentée sur la figure, le bac 25 est utilisé en outre pour ajuster la composition du liquide filtré 28, avant son recyclage dans l'unité de culture 1. En effet, la plante absorbant une partie des éléments contenus dans la solution nutritive, l'eau de drainage est en fait un liquide nutritif appauvri. Pour tirer un meilleur profit du recyclage, il est préférable de réintroduire les éléments manquants pour ajuster la composition du liquide recyclé à la composition de la solution nutritive 4. A cet effet, une conduite 30 d'amenée de liquide, munie d'une vanne 31, permet d'introduire une quantité définie de solution nutritive 4 dans le bac 25 pour la mélanger avec le liquide 28.

En variante, la correction de la composition du liquide 28 peut se faire au moyen d'une solution spécifique, la solution 4 contenue dans la réserve 11 servant alors uniquement à alimenter directement la plante 2.

En variante, l'unité de culture 1 pourrait représenter une culture de plantes en pot sur un substrat 3 organique, tel que du terreau ou de la tourbe. Dans ce type de culture, la canalisation 10 peut délivrer alternativement de l'eau pure si l'on souhaite réaliser un simple arrosage des plantes, ou de l'eau additionnée d'éléments nutritifs si l'on souhaite enrichir le milieu organique de culture.

La colonne 7 peut également être munie de moyens de vidange non représentés pour remplacer tout ou partie du matériau filtrant 18 à base de laine de verre.

La laine de verre manifeste une excellente stabilité chimique en présence des effluents constitués par les eaux de drainage horticoles, qui peuvent être très acides.

Dans le cas de la culture hors sol, comme dans celui de la culture de plantes en pot, l'effluent de drainage 5 est en effet généralement une solution acide, de pH typiquement inférieur à 6,5, le plus souvent à 6.

La culture sur terreau produit généralement des effluents dont le pH de l'ordre de 4 à 5,5 résulte de l'acidité naturelle du substrat de culture, par exemple de la tourbe. L'arrosage à l'eau de pluie peut également être en partie responsable de cette acidité.

Par ailleurs, la tendance actuelle en culture hors-sol est à utiliser des solutions nutritives de plus en plus acides. Il est récemment apparu que les rendements de culture de fleurs telles que les roses, les oeillets ou les chrysanthèmes, mais aussi de culture des concombres étaient sensiblement améliorés avec une solution nutritive de pH compris entre 4,5 et 5, au lieu du pH habituel de l'ordre de 5,5 à 6,5. Il s'agit là de valeurs moyennes et le pH peut être encore plus faible selon les stades de culture.

Ainsi, après la cueillette des fleurs, on observe une chute du pH moyen dans le substrat de culture jusqu'à des valeurs de l'ordre de 4 à 5. En outre, la variation du pH au sein d'un pain de culture peut être très spectaculaire : on a mesuré que pour un pH moyen de 5,2, le profil d'acidité dans le volume du pain est tel que le pH est compris entre 3,5 et 4,0 dans 30 % du volume et entre 3,0 et 3,5 dans une fraction pouvant aller jusqu'à 10 % du volume. Ainsi, en cas d'écoulement irrégulier, il peut arriver que le pH de l'eau de drainage collectée soit même bien inférieur à 4.

Les présents inventeurs ont montré par des essais comparatifs que la laine de verre est plus appropriée que la laine de roche pour le traitement de tels effluents : ainsi dès que le pH de l'effluent est inférieur ou égal à 5, en particulier de l'ordre de 4,5, la laine de roche a tendance à se dissoudre sur son passage. Il se forme alors des canaux par lesquels le liquide traverse rapidement la masse de laine de roche sans atteindre le degré de purification suffisant. Au contraire, la laine de verre reste intacte même dans des milieux très acides (par exemple pH de l'ordre de 4) et peut être utilisée durablement pour purifier efficacement de tels effluents.

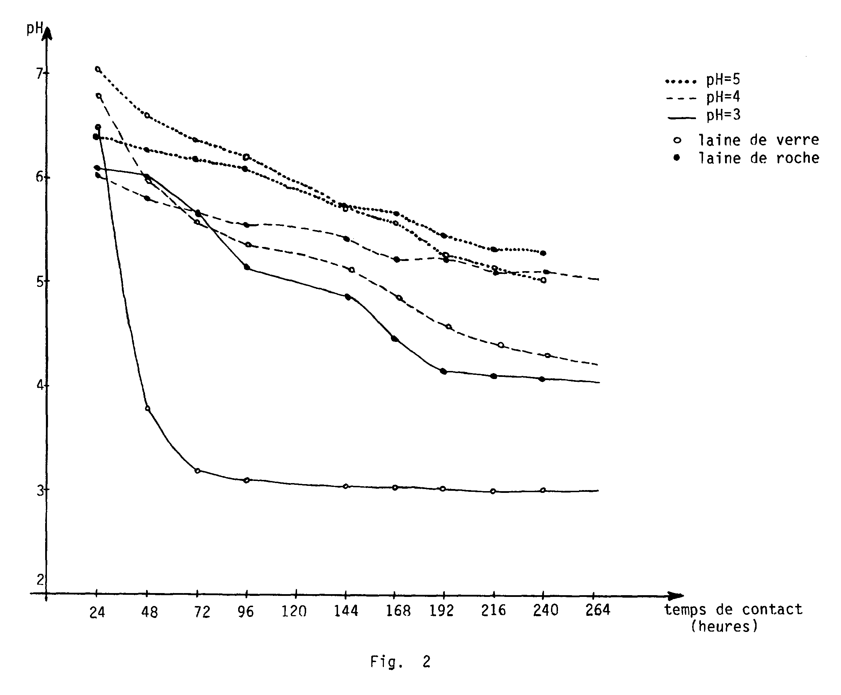

Ces essais comparatifs sont illustrés par le graphe de la figure 2.

Des échantillons de matériau fibreux de dimensions identiques sont découpés respectivement dans un pain de laine de roche de densité de l'ordre de 80 kg/m³ et dans un pain de laine de verre de densité de l'ordre de 50 kg/m³. On les met en présence d'une solution nutritive de type Kanimajor dont la conductivité est ajustée à Ec = 2 mS par de l'eau permutée et le pH est ajusté par de l'acide nitrique, en opérant de la façon suivante : chaque échantillon est placé dans un pot que l'on trempe dans de la solution nutritive jusqu'à immersion complète de l'échantillon ; on sort ensuite le pot de la solution, on recouvre l'échantillon et on laisse drainer pendant 24 heures.

Après 24 heures, on extrait la solution nutritive de l'échantillon au moyen d'une fiole à vide équipée d'un Büchner, jusqu'à ce que la solution ne s'écoule pratiquement plus dans la fiole, et l'on mesure le pH de la solution extraite.

On remet ensuite l'échantillon dans son pot et on procède à une nouvelle immersion, suivie d'un drainage de 24 h.

On recommence ainsi l'opération chaque jour pendant 2 semaines.

Pour chaque type de matériau, on a testé 3 échantillons et calculé la moyenne des 3 mesures quotidiennes avec 3 solutions nutritives respectivement de pH 5, 4 et 3.

Ces valeurs moyennes sont reportées suivant les courbes de la figure 2.

De façon générale, on observe que pendant les premiers jours du test, la solution extraite présente un pH supérieur au pH de la solution nutritive initiale.

Cette alcalinisation est un signe révélateur d'une interaction de type acido-basique entre la solution acide et les groupes fonctionnels alcalins (oxydes) du matériau fibreux.

Si dans les premières heures, cette réaction semble un peu plus marquée avec la laine de verre qu'avec la laine de roche, la situation s'inverse rapidement :
- d'une part, avec la laine de verre, le pH de la solution de contact atteint très vite une valeur d'équilibre très proche du pH nominal de la solution de contact ; l'état stable finalement atteint est un état dans lequel le matériau fibreux est inerte vis-à-vis du liquide ;
- d'autre part, avec la laine de roche, le pH de la solution de contact se stabilise plus lentement, la valeur d'équilibre étant toutefois supérieure au pH nominal de la solution nutritive, la différence étant d'autant plus grande que ce pH nominal est faible ; l'état stable finalement atteint est un état dans lequel le matériau fibreux se dégrade régulièrement au contact du liquide. Cette dégradation se manifeste par l'apparition de trous ou canaux dans le matériau.

A cette différence de propriétés chimiques, s'ajoute le fait que la laine de roche, en raison de sa technique de fabrication, renferme une forte proportion de matière infibrée de type plutôt granulaire, ce qui diminue la surface spécifique moyenne du matériau. La laine de verre, qui a une structure fibreuse plus stratifiée et plus régulière et un taux d'infibrés très faible, généralement inférieur à 5 %, se caractérise par une surface spécifique plus élevée, et permet d'obtenir une filtration de meilleure qualité et une désinfection plus efficace.

Le fonctionnement de l'installation décrite ci-dessus est illustré en outre par l'exemple suivant.

On équipe une serre expérimentale de 300 m² de culture de tomates sur substrat de laine de roche de dispositifs de recyclage tels que décrits ci-dessus, une colonne de filtration étant associée à une surface de culture de 20 m². Chaque colonne a un diamètre inférieur de 15 cm et une hauteur de 2 m environ. Le garnissage de la colonne se fait de la façon suivante : on dispose au pied de la colonne une couche de gravier de 10 cm environ. On découpe dans un matelas de laine de verre de densité 50 kg/m³ environ et de 10 cm d'épaisseur des disques de 15,5 cm de diamètre, soit d'un diamètre supérieur de 3,3 % au diamètre intérieur de la colonne. Huit disques sont successivement introduits à force dans la colonne 7 et empilés pour former une couche de 80 cm d'épaisseur. Les propriétés mécaniques du matelas de laine de verre sont telles que les disques légèrement compressés au cours de cette opération épousent parfaitement le volume intérieur de la colonne 7 sans subir aucune détérioration. Grâce à ses propriétés élastiques et à sa capacité de reprise de volume (« effet ressort »), le feutre de laine de verre est donc un matériau de garnissage avantageux qui permet de supprimer les courts circuits de liquide le long de la paroi intérieure de la colonne.

Ce garnissage laisse un espace libre d'environ 1 m dans la partie supérieure de la colonne servant de capacité de stockage d'eau de drainage. Cette capacité, utile pour imposer la force motrice dans la colonne, sert également de capacité - tampon lorsque l'alimentation en eau de drainage se fait de manière intermittente.

Parmi les pathogènes affectant les plants de tomate, on peut citer en particulier *Phytophtora cinnamomi (Pc), Fusarium oxysporum* f. sp. */ycopersci* (*Fo*), et le virus mosaïque de la tomate. En filtrant l'eau de drainage sur la laine de verre à une vitesse linéique de 0,1 m/h, ces pathogènes peuvent être éliminés à au moins 99 %, taux de désinfection qui permet de recycler sans risque le filtrat dans le circuit d'arrosage des plants de tomate. Rappelons que cette épuration est le résultat d'une simple filtration sur la laine de verre, sans recourir à un agent chimique tel que l'ozone, ou à un traitement du type absorption sur charbon actif, ou autres.

Des essais ont été pratiqués avec deux densités différentes de laine de verre, à savoir environ 50 et environ 100 kg/m³, et avec deux types de laine de verre, à savoir un matelas de laine de verre dans lequel les fibres sont revêtues et liées par un liant organique de type phénol-formaldéhyde (laine jaune) et le même matelas débarrassé de son liant par traitement thermique de pyrolyse (laine blanche).

Dans tous les cas, l'élimination de Pc est assurée à 100 %. Le degré d'élimination de Fo est indiqué qualitativement dans le tableau suivant où la note (+ + + +) qualifie une épuration au moins 2 fois meilleure que la note (+ +).

| laine | élimination Fo |
|---|---|
| d = 50 kg/m³ | + + |
| d = 100 kg/m³ | + + + + |
| laine jaune | + + + + |
| laine blanche | + + |

Par rapport à un système de filtration sur sable, le système selon l'invention présente de nombreux avantages.

Tout d'abord, la qualité de la filtration est sensiblement améliorée par la stabilité mécanique et dimensionnelle du matériau filtrant. Dans le sable, qui a une structure meuble, la circulation de l'eau de drainage s'accompagne de la formation de canaux qui court-circuitent la filtration. Au contraire les fibres de laine de verre ont tendance à s'enchevêtrer pour donner une structure résistante. Le parcours du liquide au sein du matériau filtrant est sensiblement allongé et la filtration gagne en efficacité.

Par rapport à la laine de roche, la laine de verre a une surface spécifique supérieure due à l'absence d'infibrés qui implique également un gain d'efficacité.

En outre, un inconvénient du sable réside dans le fait que, au fur et à mesure du passage de liquide sur la colonne, on observe à la surface du filtre le développement d'algues qui obstruent peu à peu les pores permettant le passage du liquide. Le nettoyage ou le remplacement de cette couche de sable de surface est délicat car le matériau est dense et des lourdes quantités de matériau granulaire sont difficiles à manipuler.

De façon surprenante, les présents inventeurs ont constaté que ce phénomène n'a pas lieu lorsque le filtre est constitué de laine de verre. La maintenance de l'installation en est considérablement simplifiée et le filtre peut fonctionner plus longtemps à efficacité optimale.

En outre, si l'on souhaite pour une raison quelconque remplacer la couche de surface ou une autre partie du filtre, l'utilisation de feutre de laine de verre, éventuellement en couches superposées, facilite cette opération et permet ainsi de gagner du temps.

Un avantage capital de l'invention est que l'on réalise des installations de désinfection beaucoup moins encombrantes avec la laine minérale qu'avec le sable. Actuellement, pour traiter les 2,5 m³ d'eau de drainage rejetés par heure par 1 hectare de culture hors-sol fonctionnant avec 30 % de drainage, par filtration lente sur sable à une vitesse linéique de 0,1 m/h, il faut un filtre de 25 m² de surface sur 1 m de haut. En outre, cette technique ne permet d'arrêter que les gros pathogènes tels que *Phytophthora* et *Pythium,* mais les autres pathogènes tels que *Fusarium* et les virus ne sont pas éliminés correctement.

Suivant la présente invention, on parvient à une désinfection satisfaisante dans les mêmes conditions avec seulement 8 à 12 m³ de laine de verre, grâce à la surface spécifique élevée de ce matériau.

Enfin, comme on l'a évoqué précédemment, un autre avantage essentiel de l'invention est qu'elle permet de traiter des effluents acides dont le pH peut être très faible et atteindre des valeurs de l'ordre de 3, alors que les systèmes existants à base de laine de roche se dégradent irrémédiablement même avec des effluents traditionnels essentiellement neutres dès que se produit une chute accidentelle de pH de l'effluent.

## Revendications

1. Procédé de désinfection d'un effluent aqueux acide dont le pH peut notamment atteindre des valeurs de l'ordre de 3 à 5, notamment de l'eau de drainage d'une culture hors-sol ou d'une culture de plante en pot qui renferme des micro-organismes pathogènes de ladite culture, en vue de son recyclage en tant que liquide d'arrosage et/ou nutritif pour ladite culture, **caractérisé en ce que** l'étape de désinfection consiste à faire circuler l'effluent à travers une masse de matériau filtrant à base de laine de verre.

2. Procédé selon la revendication 1, **caractérisé en ce que** l'effluent aqueux a un pH inférieur à 5, notamment de l'ordre de 4 à 4,5.

3. Procédé selon la revendication 2, **caractérisé en ce que** la laine de verre présente un taux d'infibrés inférieur à 5 %.

4. Procédé selon l'une quelconque des revendications précédentes, **caractérisé en ce que** l'effluent circule à travers le matériau filtrant à une vitesse linéique inférieure à 0,3 m/h, de préférence à une vitesse linéique d'environ 0,1 m/h.

5. Procédé selon l'une quelconque des revendications précédentes, **caractérisé en ce que** le matériau filtrant a une surface spécifique d'au moins 0,01 m²/cm³.

6. Procédé selon l'une quelconque des revendications précédentes, **caractérisé en ce que** la laine de verre a une masse volumique de l'ordre de 30 à 250 kg/m³, notamment d'environ 50 à 100 kg/m³.

7. Procédé selon l'une quelconque des revendications précédentes, **caractérisé en ce que** la laine de verre a un micronaire inférieur à 7 pour 5 grammes, notamment de l'ordre de 1 à 5.

8. Procédé selon l'une quelconque des revendications précédentes, **caractérisé en ce qu'**au moins une partie du matériau filtrant est constituée de laine de verre sous forme de feutre.

9. Procédé selon l'une quelconque des revendications précédentes, **caractérisé en ce qu**'au moins une partie de la laine de verre est enduite d'un liant organique de type phénol formaldéhyde.

10. Procédé selon l'une quelconque des revendications précédentes, **caractérisé en ce qu**'au moins une partie du matériau filtrant renferme un agent mouillant.

11. Procédé de recyclage d'une eau de drainage acide d'une culture hors-sol ou d'une culture de plante en pot équipée d'un circuit d'arrosage et/ou d'alimentation en solution nutritive, **caractérisé en ce qu**'il comprend les étapes consistant à :
- recueillir l'eau de drainage à partir du milieu de culture,
- soumettre l'eau recueillie à un procédé de désinfection sur un matériau filtrant selon l'une quelconque des revendications 1 à 10,
- introduire l'effluent désinfecté qui s'écoule hors du matériau filtrant dans le milieu de culture en tant que liquide d'arrosage et/ou d'alimentation en liquide nutritif.

12. Dispositif pour la mise en oeuvre d'un procédé de désinfection d'un effluent aqueux acide (5) selon l'une quelconque des revendications 1 à 10, **caractérisé en ce qu**'il comprend :
- une capacité (15, 21) de stockage d'un effluent acide, notamment de l'eau de drainage d'une culture hors-sol ou d'une culture de plante en pot qui renferme des micro-organismes pathogènes de ladite culture,
- une masse (18) de matériau filtrant à base de laine de verre avec une extrémité d'entrée (19) alimentée en effluent aqueux (5) par des moyens d'amenée de liquide (17, 16) reliés à la capacité de stockage (15) et une extrémité de sortie (20) délivrant un effluent désinfecté (29), et
- des moyens (21) pour faire circuler l'effluent (5) entre lesdites extrémités d'entrée (19) et de sortie (20).

13. Dispositif selon la revendication 12, **caractérisé en ce que** ladite extrémité d'entrée (19) est située dans la partie supérieure du matériau filtrant (18) et ladite extrémité de sortie (20) est située dans la partie inférieure du matériau filtrant (18).

14. Dispositif selon la revendication 13, **caractérisé en ce que** le matériau filtrant (18) comprend une partie supérieure (22) où la laine de verre est sous forme de feutre, une partie intermédiaire (23), et une partie inférieure (24) éventuellement constituée d'un autre matériau poreux.

15. Dispositif selon l'une quelconque des revendications 12 à 14, **caractérisé en ce que** lesdits moyens pour faire circuler l'effluent entre les extrémités d'entrée (19) et de sortie (20) du matériau filtrant (18) comprennent une capacité (21) remplie dudit effluent et qui surmonte le matériau filtrant (18).

16. Dispositif de recyclage d'une eau de drainage acide (5) d'une culture hors-sol ou d'une culture de plante en pot (1) équipée d'un circuit (10) d'arrosage et/ou d'alimentation en solution nutritive (4), **caractérisé en ce qu**'il comprend :
- des moyens (13, 14) pour recueillir ladite eau de drainage (5), reliés à des premiers moyens (16, 17) d'amenée de liquide,
- un dispositif de désinfection (6) selon l'une quelconque des revendications 12 à 15, dont la capacité de stockage est alimentée en effluent liquide (5) par lesdits premiers moyens (16, 17) d'amenée de liquide, et
- des seconds moyens (8, 30, 9) d'amenée de liquide reliant l'extrémité de sortie (20) du matériau filtrant (18) audit circuit d'arrosage et/ou d'alimentation en solution nutritive.

17. Filtre (18) pour un dispositif (6) de traitement d'un effluent aqueux, selon l'une quelconque des revendications 12 à 16, **caractérisé en ce qu**'il est à base de laine de verre et en ce qu'il comprend une partie supérieure (22) où la laine de verre est sous forme de feutre, une partie intermédiaire (23), et une partie inférieure (24) éventuellement constituée d'un autre matériau poreux.
